# EUROPEAN PATENT APPLICATION

(11) **EP 4 520 370 A1**
(43) Date of publication of application: **12.03.2025**
(21) Application number: 23865650.8
(22) Date of filing: 02.06.2023
(51) Int. Cl.: A61M 15/00, A24F 40/20, A24F 40/42, A24F 40/40

(54) **INHALER**

(30) Priority: 14.09.2022 KR 20220115597
(71) Applicant: KT&G Corporation, Daedeok-gu Daejeon 34337 (KR)
(72) Inventor: JUNG, Yongmi, Daejeon 34128 (KR); KIM, Moonwon, Daejeon 34128 (KR); SHIN, Jun Won, Daejeon 34128 (KR)
(74) Representative: Ter Meer Steinmeister & Partner
(86) International application number: PCT/KR2023/007602
(87) International publication number: WO 2024/058346

(57) **Abstract**

An inhaler for inhaling functional materials according to various embodiments may comprise: a holder comprising an opening and an inner space that is in communication with the opening; a piercing member which is arranged in the inner space and protrudes toward the opening; and a functional material accommodation member which is inserted from the opening into the inner space of the holder along the lengthwise shaft of the holder and accommodates the functional material, wherein the piercing member may include a plurality of holes which are air-permeable with each other.

## Description

### Technical Field

The following embodiments relate to an inhaler.

### Background Art

Research has been conducted into an inhaler for delivering target materials directly to the lungs of a user. For example, KR Patent Application Publication No. 10-2016-0065204 discloses a dry powder inhaler.

### Disclosure of the Invention

### Technical Goals

An inhaler according to various embodiments may induce vortex movement of functional material powder, even if a stick does not have a separate vortex tunnel structure.

An inhaler according to various embodiments may induce smooth discharge of functional material powder.

An inhaler according to various embodiments may prevent noise caused by rotation of a capsule.

An inhaler according to various embodiments may prevent leakage of functional material powder from the bottom of a capsule by preventing a bottom hole of the capsule from being exposed.

An inhaler according to various embodiments may provide an appropriate inhalation resistance.

### Technical Solutions

An inhaler according to various embodiments includes a holder including an opening and an inner space communicating with the opening, a piercing member that is arranged in the inner space and protrudes toward the opening, and a functional material accommodation member that is inserted from the opening into the inner space of the holder along a longitudinal axis of the holder and is configured to accommodate the functional materials, wherein the piercing member may include a plurality of holes through which air flows through each other.

In an embodiment, the holder may include a first holder surface in which the opening is formed, a second holder surface facing the first holder surface, and a side holder surface between the first holder surface and the second holder surface, wherein the inner space may include a recess formed concave from the opening towards the second holder surface, and wherein the piercing member may protrude from an end of the inner space toward the opening.

In an embodiment, the piercing member may include a base combined with the end of the inner space, a body extending from the base towards the opening, a tip arranged at an end of the body, an airflow path formed inside the body, and a plurality of airflow holes that communicates with the airflow path and is formed on an outside of the body.

The plurality of airflow holes may be formed to be spaced apart along a longitudinal direction of the body.

In an embodiment, the functional material accommodation member may include stick that is insertable into the inner space and a capsule that is arranged inside the stick and is configured to accommodate the functional materials.

A length from the base to the tip of the piercing member may be configured to be longer than a longitudinal length of the capsule.

In an embodiment, the inhaler may further include an elastic member arranged at the end of the inner space.

The airflow holes may include a first airflow hole set and a second airflow hole set, wherein the first airflow hole set may be formed in a first body area of the piercing member and the second airflow hole set may be formed in a second body area of the piercing member, wherein the first body area may be an area between the base and an end of the elastic member, in a state where the elastic member is uncompressed, and wherein the second body area may be an area between the end of the elastic member and the tip, in a state where the elastic member is uncompressed.

A longitudinal length of the second body area may be configured to be shorter than the longitudinal length of the capsule.

An inlet, through which airflow flows in, may be formed between an outside of the holder and the inner space.

### Effects

According to various embodiments, vortex movement of functional material powder may be induced, even if a stick does not have a separate vortex tunnel structure.

According to various embodiments, smooth discharge of functional material powder may be achieved, as internal airflow flowed into an inhaler penetrates through the center of a functional material accommodation member.

According to various embodiments, since functional material powder may smoothly shift even if a capsule does not rotate, noise caused by rotation of the capsule may be prevented.

According to various embodiments, leakage of functional material powder from the bottom of a capsule may be prevented by preventing a bottom hole of the capsule from being exposed.

According to various embodiments, the feeling of smoking may be improved by providing an appropriate inhalation resistance due to a narrow airflow hole.

The effects of an inhaler according to various embodiments are not limited to the above-mentioned effects, and other unmentioned effects can be clearly understood from the following description by one of ordinary skill in the art.

### Brief Description of Drawings

FIG. 1 is a diagram illustrating an inhaler according to an embodiment.
FIG. 2 illustrates a configuration of an inhaler according to an embodiment.
FIG. 3 illustrates a piercing member according to an embodiment.
FIG. 4 illustrates a functional material accommodation member according to an embodiment.
FIG. 5 illustrates a configuration of a piercing member and a capsule according to an embodiment.
FIG. 6 illustrates an operation state of an inhaler according to an embodiment.

### Best Mode for Carrying Out the Invention

The terms used in the embodiments are selected from among common terms that are currently widely used, in consideration of their function in the embodiments. However, the terms may become different according to an intention of one of ordinary skill in the art, a precedent, or the advent of new technology. Also, in particular cases, the terms are discretionally selected by the applicant of the disclosure, and the meaning of those terms will be described in detail in the corresponding part of the detailed description. Therefore, the terms used in the disclosure are not merely designations of the terms, but the terms are defined based on the meaning of the terms and content throughout the disclosure.

It will be understood that when a certain part "includes" a certain component, the part does not exclude another component but may further include another component, unless the context clearly dictates otherwise. Also, terms such as "unit," "module," etc., as used in the specification may refer to a part for processing at least one function or operation and may be implemented as hardware, software, or a combination of hardware and software.

As used herein, an expression such as "at least one of" that precedes listed components modifies not each of the listed components but all the components. For example, the expression "at least one of a, b, or c" should be construed as including a, b, c, a and b, a and c, b and c, or a, b, and c.

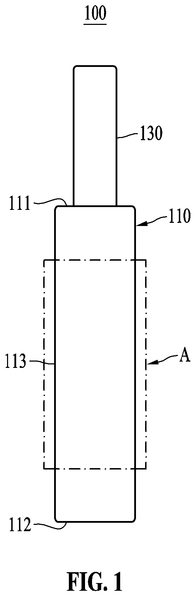

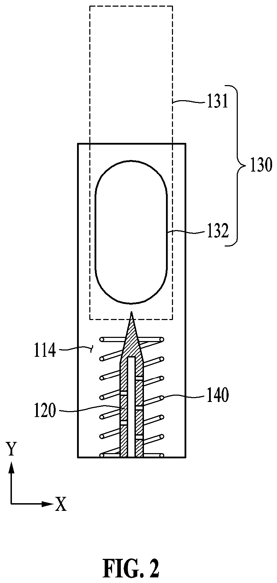

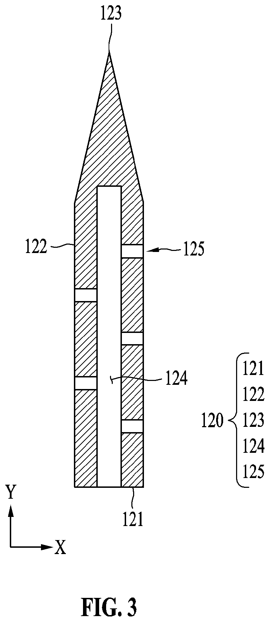

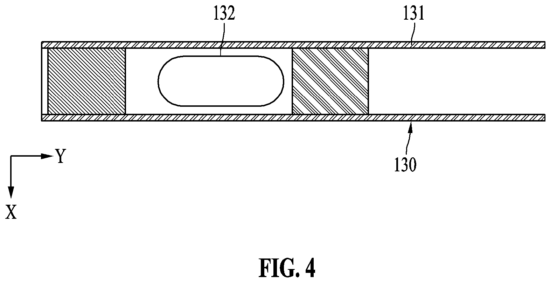

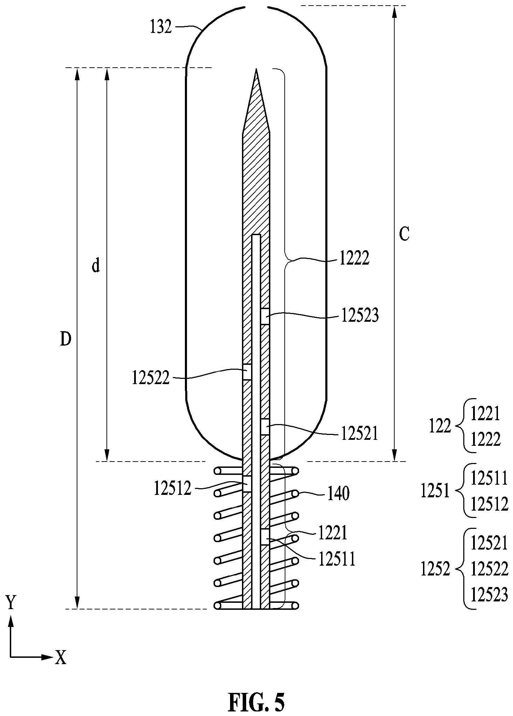

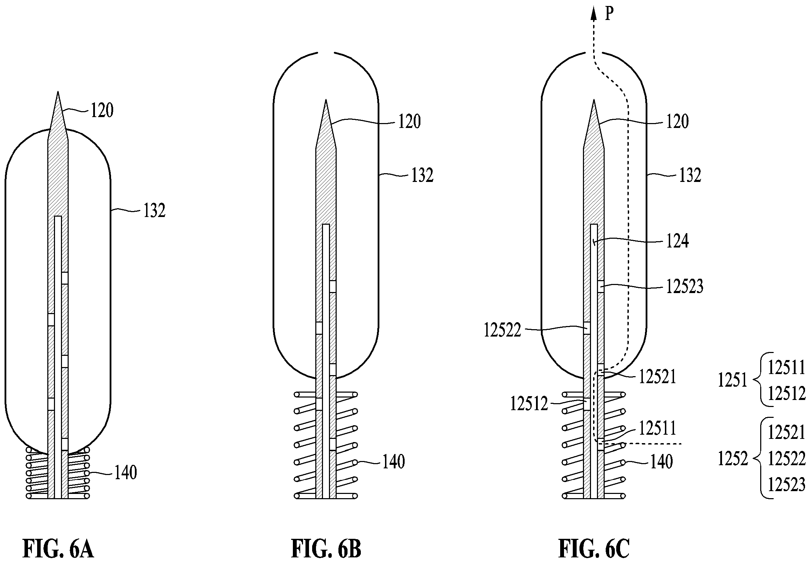

FIG. 1 illustrates an inhaler 100 according to an embodiment, FIG. 2 schematically illustrates a structure of the inhaler 100 according to an embodiment in a portion A of FIG. 1, FIG. 3 illustrates a piercing member 120 of the inhaler 100 according to an embodiment, FIG. 4 illustrates a functional material accommodation member 130 of the inhaler 100 according to an embodiment, and FIG. 5 schematically illustrates an arrangement relationship between a piercing member 120 and a capsule 132 of the inhaler 100 according to an embodiment. In addition, FIG. 6 illustrates an operation state of the inhaler 100 according to an embodiment.

According to an embodiment, the inhaler 100 may deliver functional materials to a user and for example, the inhaler 100 according to an embodiment may include an inhaler for delivering nicotine to the lungs of a user as an aerosol.

Referring to FIGS. 1 and 2, the inhaler 100 according to an embodiment may include a holder 110, the piercing member 120, and the functional material accommodation member 130.

In an embodiment, the holder 110 may be configured in a cylindrical shape having an inner space 114, and the inner spaces 114 may be formed in a size in which the functional material accommodation member 130 described below may be inserted. An opening for inserting the functional material accommodation member 130 may be formed in one side of the holder 110, and the opening and the inner space 114 are connected to each other.

In an example, the holder 110 may include a first holder surface 111, a second holder surface 112, and a side holder surface 113. The opening may be formed in the first holder surface 111, and the second holder surface 112 may be a surface formed on an opposite side of the first holder surface 111. The side holder surface 113 may be formed between the first holder surface 111 and the second holder surface 112. The inner space 114 may include a recess formed concave from the opening of the first holder surface 111 towards the second holder surface 112 and may be formed lengthwise along the longitudinal axis (e.g., ±Y direction of FIG. 2) of the holder 110.

In an embodiment, an inlet (not shown) may be formed between the outside of the holder 110 and the inner space 114 to allow air from outside the holder 110 to flow into the inner space 114.

In an embodiment, the piercing member 120 may be arranged in the inner space 114 and may be formed to protrude toward the opening of the first holder surface 111. For example, the piercing member 120 may protrude from the end of the inner space 114 in a direction toward the opening (e.g., +Y direction of FIG. 2).

The inhaler 100 according to an embodiment may further include an elastic member 140. For example, the elastic member 140 may be arranged at the end of the inner space 114 and may include a coil spring that may apply an elastic force to push the functional material accommodation member 130 in the direction toward the opening of the holder 110 (e.g., +Y direction of FIG. 2) when the functional material accommodation member 130 is inserted into the inner space 114 of the holder 110.

Referring to FIG. 3, the piercing member may include a base 121, a body 122, a tip 123, an airflow path 124, and airflow holes 125.

In an embodiment, the base 121 may be fixed to the end of the inner space 114 of the holder 110. The body 122 may extend from the base 121 along a longitudinal direction (e.g., ±Y direction of FIG. 3) toward the opening of the holder 110. The tip 123 may be arranged at the end of the body 122.

In an embodiment, an airflow path 124 may be formed inside the body 122, and for example, the airflow path 124 may be a cavity formed along the longitudinal direction from the base 121 through the body 122 to just before the tip 123.

The airflow hole 125 may be formed on the outside of the body 122 and may be a hole through which air may communicate with the airflow path 124. A plurality of airflow holes 125 may be provided, and the plurality of airflow holes 125 may be arranged apart from each other along the longitudinal direction. For example, the plurality of airflow holes 125 may be arranged apart from each other in a zig-zag manner along the longitudinal direction.

Here, one end (e.g., a -Y direction side of FIG. 3) of the airflow path 124 adjacent to the base 121 or the other end (e.g., a +Y direction side of FIG. 3) of the airflow path 124 adjacent to the tip 123 may be formed in a closed structure, and the airflow flowing in through an airflow hole 125 adjacent to the one end (e.g., the -Y direction side of FIG. 3) of the airflow path 124 may pass through the airflow path 124 and may exit into another airflow hole 125 adjacent to the other end (e.g., the +Y direction side of FIG. 3) of the airflow path 124 adjacent to the tip 123.

Referring to FIGS. 2 and 4, the functional material accommodation member 130 may include a stick 131 and a capsule 132. For example, the stick 131 may be configured in a cylindrical shape insertable into the inner space 114 of the holder 110 and the capsule 132 may be arranged inside the stick 131.

In an example, functional materials may be accommodated in the capsule 132. For example, the functional materials may include at least one of nicotine, theanine, caffeine, taurine, pharmacological materials, or mixtures thereof. The functional materials may be in the form of fine granules or dry powder.

In an example, the outer shell of the capsule 132 may include a material that may be pierced through or cut by the piercing member 120.

Particularly, referring to FIG. 4, the stick 131 of the functional material accommodation member 130 may include a plurality of sections. For example, a section including the capsule 132 may include a cavity in which the capsule is accommodated. Here, since the capsule 132 is fixed within the cavity, free rotation of the capsule 132 may be prevented and noise generation due to rotation of the capsule 132 may be prevented when the functional materials are inhaled.

As an example, a front end (e.g., a -Y direction side of FIG. 4) of the section including the capsule 132 may be provided with a front end made of cellulose acetate so that leakage of functional material powder may be prevented. A rear end (e.g., a +Y direction side of FIG. 4) of the section including the capsule 132 may be provided with a mouthpiece that may contact the mouth of a user. The cavity including the capsule 132 and the mouthpiece may be connected such that air may communicate.

The stick 131 of the functional material accommodation member 130 may not be provided with a vortex tunnel (e.g., an airflow tunnel formed on one side of the stick 131, through which air may communicate with the cavity including the capsule 132) to form a vortex. This is because airflow passing through the airflow path 124 and the airflow holes 125 of the piercing member 120 according to an embodiment may flow into the capsule 132 and may form a vortex.

Referring to FIG. 5, in an embodiment, the body 122 of the piercing member 120 may include a first body area 1221 and a second body area 1222. The first body area 1221 may be defined as an area of the body 122 between the base 121 of the piercing member 120 and the end of the elastic member 140 in a state where the elastic member 140 is not compressed. The second body area 1222 may be defined as an area of the body 122 between the end of the elastic member 140 and the tip 123 of the piercing member 120 in a state where the elastic member 140 is not compressed.

The airflow holes 125 may include a first airflow hole set 1251 and a second airflow hole set 1252. Airflow holes included in the first airflow hole set 1251 may be formed in the first body area 1221 and airflow holes included in the second airflow hole set 1252 may be formed in the second body area 1222.

For example, the airflow holes 125 may include a first airflow hole 12511, a second airflow hole 12512, a third airflow hole 12521, a fourth airflow hole 12522, and a fifth airflow hole 12523 in order from the base 121 of the piercing member 120 to the tip 123. The first airflow hole 12511 and the second airflow hole 12512 may be formed in the first body area 1221. The third airflow hole 12521, the fourth airflow hole 12522, and the fifth airflow hole 12523 may be formed in the second body area 1222. The first airflow hole set 1251 may include the first airflow hole 12511 and the second airflow hole 12512 and the second airflow hole set 1252 may include the third airflow hole 12521, the fourth airflow hole 12522, and the fifth airflow hole 12523. Here, the first airflow hole set 1251 may act as an inlet for air to flow into the airflow path 124, when the functional materials are transferred, and the second airflow hole set 1252 may act as an outlet for air to flow out to the capsule 132.

In an embodiment, the length D from the base 121 to the tip 123 of the piercing member 120 may be configured to be longer than the length C in the longitudinal direction (e.g., ±Y direction of FIG. 5) of the capsule 132. Since the length D of the piercing member 120 is sufficiently longer than the longitudinal length C of the capsule 132, the piercing member 120 may enter one side of the capsule 132 (e.g., a -Y direction side of FIG. 5) and may penetrate the other side of the capsule 132 (e.g., a +Y direction side of FIG. 5). Since the piercing member 120 penetrates the capsule 132, a through hole through which airflow may pass may be formed on the other side of the capsule 132 and the through hole may be connected to the mouthpiece of the functional material accommodation member 130 so that air may communicate.

In an embodiment, the length d of the second body area 1222 of the piercing member 120 in the longitudinal direction (e.g., ±Y direction of FIG. 5) may be configured to be shorter than the longitudinal length C of the capsule 132. Since the longitudinal length d of the second body area 1222 is configured to be shorter than the longitudinal length C of the capsule 132, when the capsule 132 is retracted from the base 121 of the piercing member 120 due to the elastic force of the elastic member 140, at least a portion of the second body area 1222 may be placed inside the capsule 132. Here, at least a portion of the airflow holes 12521, 12522, and 12523 of the second airflow hole set 1252 formed in the second body area 1222 may be located inside the capsule 132 and the airflow path 124 and the inside of the capsule 132 may be connected so that air may communicate.

In another example, when the inhaler 100 according to an embodiment is not provided with a separate elastic member, a user may insert the stick 131 of the functional material accommodation member 130 into the inner space 114 of the holder 110, may press the stick 131 appropriately, and then may lift the stick 131 slightly while the stick 131 is inserted into the inner space 114. Here, the user may lift the stick 131 only to an inhalation position so that the second airflow hole set 1252 is placed inside the capsule 132. A marker (e.g., a guideline) indicating the inhalation position may be displayed on the outside surface of the holder 110 or the stick 131.

Hereinafter, an operation state of the inhaler 100 according to an embodiment is described with reference to FIG. 6.

Referring to (a) of FIG. 6, when the functional material accommodation member 130 is inserted into the holder 110, the capsule 132 may be penetrated in the longitudinal direction by the piercing member 120. Here, when the inhaler 100 according to an embodiment includes an elastic member 140, the elastic member 140 may be compressed by the functional material accommodation member 130.

Referring to (b) of FIG. 6, the capsule 132 may retract from the base 121 of the piercing member 120 and functional materials may be placed in the inhalation position. Here, a through hole may be formed at one end of the capsule 132 due to the retraction of the piercing member 120 and the through hole may be connected to the inner space 114 of the holder 110, the airflow hole 125, the airflow path 124, and the inner space of the capsule 132 in a state where air may communicate.

For example, when the inhaler 100 according to an embodiment includes the elastic member 140, the capsule 132 may retract due to the restoring force (elastic force) of the elastic member 140. In another example, when the elastic member 140 is not provided, a user may move the capsule 132 to the inhalation position using a marker indicating the inhalation position.

Referring to (c) of FIG. 6, an airflow path P directed toward the inner space 114 of the holder 110, the first airflow hole set 1251, the airflow path 124, the second airflow hole set 1252, the inner space of the capsule 132, and the mouthpiece may be formed when the user applies inhalation force while the user holds the mouthpiece at the inhalation position.

Here, airflow (air) flowing into the airflow path 124 through the first airflow hole 12511 or the second airflow hole 12512 may flow into the inner space of the capsule 132 through the third airflow hole 12521, the fourth airflow hole 12522, or the fifth airflow hole 12523. Since the first airflow hole 12511, the second airflow hole 12512, the third airflow hole 12521, the fourth airflow hole 12522, or the fifth airflow hole 12523 are arranged to be spaced apart in a zigzag manner in the longitudinal direction, the airflow flowing into the inner space of the capsule 132 may form a vortex. Due to the vortex, functional materials (e.g., nicotine powder) may smoothly move towards the mouthpiece even if the capsule 132 does not rotate.

According to the inhaler 100 according to an embodiment, even if a separate vortex tunnel is not formed in the stick 131, the airflow flowing into the inner space of the capsule 132 may form a vortex due to the configuration of the airflow hole 125 formed in the piercing member 120 and accordingly, the movement of the functional materials (e.g., nicotine powder) may be facilitated.

In addition, according to the inhaler 100 according to an embodiment, even if the capsule 132 does not rotate, functional materials may smoothly move due to the configuration of the airflow hole 125 formed in the piercing member 120, and thus, unnecessary noise caused by rotation of the capsule 132 may be prevented.

Since functional materials are inhaled in a state where the piercing member 120 of the inhaler 100 according to an embodiment is inserted at the bottom of the capsule 132, unnecessary leakage of functional powder through the bottom of the capsule 132 may be reduced.

Since the airflow path P of the inhaler 100 according to an embodiment includes relatively narrow airflow holes 125, the airflow holes 125 may provide appropriate inhalation resistance. Accordingly, a better smoking feeling may be provided to a user.

The descriptions of the above-described embodiments are merely examples, and it will be understood by one of ordinary skill in the art that various changes and equivalents may be made thereto. Therefore, the scope of the disclosure should be defined by the appended claims, and all differences within the scope equivalent to those described in the claims will be construed as being included in the scope of protection defined by the claims.

## Claims

1. An inhaler for inhaling functional materials, the inhaler comprising:
a holder comprising an opening and an inner space communicating with the opening;
a piercing member that is arranged in the inner space and protrudes toward the opening; and
a functional material accommodation member that is inserted from the opening into the inner space of the holder along a longitudinal axis of the holder and is configured to accommodate the functional materials,
wherein the piercing member comprises a plurality of holes through which air communicates.

2. The inhaler of claim 1, wherein
the holder comprises a first holder surface in which the opening is formed, a second holder surface facing the first holder surface, and a side holder surface between the first holder surface and the second holder surface, wherein the inner space comprises a recess formed concave from the opening towards the second holder surface, and
wherein the piercing member protrudes from an end of the inner space toward the opening.

3. The inhaler of claim 2, wherein
the piercing member comprises:
a base combined with the end of the inner space;
a body extending from the base towards the opening;
a tip arranged at an end of the body;
an airflow path formed inside the body; and
a plurality of airflow holes that communicates with the airflow path and is formed on an outside of the body.

4. The inhaler of claim 3, wherein
the plurality of airflow holes is formed to be spaced apart along a longitudinal direction of the body.

5. The inhaler of claim 4, wherein
the functional material accommodation member comprises:
a stick that is insertable into the inner space; and
a capsule that is arranged inside the stick and is configured to accommodate the functional materials.

6. The inhaler of claim 5, wherein
a length from the base to the tip of the piercing member is configured to be longer than a longitudinal length of the capsule.

7. The inhaler of any one of claims 1 to 6, further comprising:
an elastic member arranged at the end of the inner space.

8. The inhaler of claim 7, wherein
the airflow holes include a first airflow hole set and a second airflow hole set, wherein the first airflow hole set is formed in a first body area of the piercing member and the second airflow hole set is formed in a second body area of the piercing member,
wherein the first body area is an area between the base and an end of the elastic member, in a state where the elastic member is uncompressed, and wherein the second body area is an area between the end of the elastic member and the tip, in a state where the elastic member is uncompressed.

9. The inhaler of claim 8, wherein
a longitudinal length of the second body area is configured to be shorter than the longitudinal length of the capsule.

10. The inhaler of claim 1, wherein
an inlet, through which airflow flows in, is formed between an outside of the holder and the inner space.
